# EUROPEAN PATENT SPECIFICATION

(11) **EP 3 858 433 B1**
(45) Date of publication and mention of the grant of the patent: **11.10.2023**
(21) Application number: 19867225.5
(22) Date of filing: 26.02.2019
(51) Int. Cl.: A61N 2/04, G06T 7/33

(54) **TMS POSITIONING NAVIGATION APPARATUS FOR TRANSCRANIAL MAGNETIC STIMULATION TREATMENT**
NAVIGATIONSVORRICHTUNG ZUR TMS-POSITIONIERUNG FÜR EINE TRANSKRANIELLE MAGNETISTIMULATIONSBEHANDLUNG
DISPOSITIF DE NAVIGATION ET POSITIONNEMENT POUR UN TRAITEMENT DE STIMULATION MAGNÉTIQUE TRANSCRÂNIENNE

(30) Priority: 27.09.2018 CN 201811131181
(43) Date of publication of application: 04.08.2021
(73) Proprietor: Wuhan Znion Technology Co., Ltd, Wuhan, Hubei 430000 (CN)
(72) Inventor: SUN, Cong, Wuhan, Hubei 430000 (CN); WANG, Bo, Wuhan, Hubei 430000 (CN); CAI, Sheng'an, Wuhan, Hubei 430000 (CN)
(74) Representative: Grünecker Patent- und Rechtsanwälte PartG mbB
(86) International application number: PCT/CN2019/076099
(87) International publication number: WO 2020/062773

(56) References cited:
- CN-A- 103 558 910
- CN-A- 106 110 507
- US-A1- 2013 085 316
- US-A1- 2013 345 491
- US-A1- 2017 128 737
- US-A1- 2017 372 006

## Description

### Technical Field

The present invention belongs to the field of TMS medical technologies, specifically, to a TMS positioning and navigation apparatus for TMS treatment.

### Background

According to statistics from the Mental Health Center of the Chinese Center for Disease Control and Prevention, the total number of patients with mental illness in China has exceeded 100 million at present, but the public's awareness of mental illness is less than 50%, and the consultation rate is even lower. At present, about 20% of the patients with mental illness receive timely treatment, and 80% of the patients with mental illness are not treated in time, or even the most basic treatment. The number of patients with severe mental illness is as high as 16 million. According to the latest statistical data from the IMS Health, the global consumption of drugs for mental illness have exceeded 36 billion U.S. dollars, accounting for 5% of the total drug sales. However, as far as China is concerned, the current mental illness drug market is still relatively small, accounting for about 1.5% of the total hospital sales. There are already more than 600 psychiatric hospitals in China, but compared with the increasing incidence of mental illness, there is still a great gap to the needs of patients with mental illness in quantity and quality. There are still a large number of patients with mental illness who cannot get professional, systematic, and effective treatment.

TMS is a technique to generate an electric current in the local cerebral cortex by a pulsed magnetic field to temporarily activate or inhibit the cortex. In the current field of medical devices, the operation of a TMS treatment device is to control a TMS coil by manual operation or by fixing same using a support to treat a patient. Manual operation is very inconvenient, and the coil needs to be held by hand for a long time or needs to be fixed at a specific angle by a support. The patient does not have good experience, because the patient needs to sit and keep a posture and dare not move. Repositioning is required after the patient moves. Manual positioning is complicated and not accurate enough, so that a treatment effect of the patient is greatly reduced.

The Chinese patent CN107149722 with application number 201710467812.0 discloses a TMS treatment device, including a TMS coil, a support, a mechanical arm, a controller, and a positioning means. The positioning means detects positions of a human head and a TMS coil, and sends the positions to the controller, and the controller controls six driving mechanisms of the mechanical arm to rotate corresponding angles. Because the mechanical arm has six degrees of freedom, the TMS coil can stimulation of the entire brain region. However, the positioning means in this patent adopts two infrared camera and one processor, and position information obtained thereby is not precise enough, so that a specific part of the head cannot be effectively treated, and thus the treatment effect will be much reduced. In addition, this positioning means does not have a repeated positioning function. A same patient needs to be repositioned every time the patient goes for treatment, thereby reducing treatment efficiency. Further improvement is urgently needed.

US 2013/085316 A1 discloses an apparatus for computer-aided, robotic delivery of transcranial magnetic stimulation (TMS) using biologically derived feedback to establish coil position relative to brain functional regions. The apparatus includes a TMS coil mounted to a robotic member. The position of the stimulating coil can be automatically optimized using the TMS-induced bio-responses of various types.

US 2017/128737 A1 discloses an operation teaching device to adjust an object to a predefined position and direction, including: a TOF type depth image camera for obtaining 3D shape information about the object; extraction means for extracting a feature region from the 3D shape information; and generation means for calculating a deviation between the 3D shape information.

US 2017/372006 A1 discloses a treatment system which may generate a fitted head model using a predetermined head model and a plurality of points. The plurality of points may include facial feature information and may be determined using a sensor, for example, an IR or optical sensor. One or more anatomical landmarks may be determined and registered in association with the fitted head model using the facial feature information.

CN 106 110 507 A discloses a navigation positioning device for a transcranial magnetic stimulator. A positioning method for the device comprises the steps: building a head model; collecting and processing an image; calculating the coordinates of a target spot; carrying out the coil positioning; carrying out the repeated positioning; carrying out the stimulation treatment; and enabling the coil to return to a standby position after stimulation.

US 2013/345491 discloses an image data processing device for TMS treatment. The device is provided with: a storage means to store a 3D MRI image of a subject's head; a 3D appearance image generation means to generate a 3D appearance image of the head; an image generation means to generate a 3D image of the subject's head.

CN 103 558 910 A discloses a display system for automatically tracking a head posture. The system can capture the head posture of a human body in real time, and a display follows the head of the human body.

It is the object of the present invention to provide an improved TMS positioning and navigation method and apparatus for TMS treatment.

This object is solved by the subject matter of the independent claims.

Embodiments are defined in the dependent claims.

The objective of the present invention is to provide, for the problem existing in the prior art, a TMS positioning and navigation method for TMS treatment. An RGBD image, an infrared image, and a depth image of a patient face can be effectively obtained through a three-dimensional (3D) camera, and spatial positions of patient face feature points are obtained based on these images, to provide data support for precise positioning of a pose of a patient head, a movement path of a mechanical arm is reasonably planned, so that the mechanical arm can automatically move a TMS coil to a magnetic stimulation point of the patient head for treatment, thereby reducing the burden on a doctor, and also improving treatment efficiency and a treatment effect. The problem in the prior art of imprecise positioning of the patient head by only two infrared cameras and one processing module is solved.

To achieve the above objective, the present invention is as defined in the appended claims. Embodiments, examples or aspects in the following disclosure, in particular methods, which do not fall under the scope of the claims are presented for illustration purposes only and do not form part of the invention.

Disclosed herein is a TMS positioning and navigation method for TMS treatment, comprising the following steps:
S1, making a patient lie flat on a horizontal translation platform, translating the horizontal translation platform to a predetermined position, and turning on a TMS treatment system by an operator;
S2, acquiring RGBD image data of a patient face by adopting a 3D camera, and obtaining stable RGBD image data and 3D point cloud image data through calibration, background removal, filtering, and an illumination compensation algorithm;
S3, adopting a face detection algorithm for an RGBD image of the patient face to obtain a patient face region, and then obtaining patient face feature point information through an ASM feature point detection algorithm;
S4, spatially positioning the patient face, establishing a space coordinate system by taking a 3D camera as an origin point, and obtaining 3D coordinate values of patient face feature points in a 3D camera space coordinate system according to the 3D point cloud image data;
S5, modeling the patient head, rotating the 3D camera around the head at a uniform speed or performing photographing at the same time by using multiple cameras to obtain image data of the patient head in all directions, by identifying the face feature point information in the image data in all the directions, calculating matching relationships between images, then obtaining a spatial position relationship between point clouds through an ICP algorithm of the 3D point clouds, integrating all the 3D point cloud image data in scan data to obtain complete 3D data of the patient head, then by taking MNI brain space coordinates commonly used in medicine as a standard, mapping 3D skull data of an MNI space obtained by brain 3D scanning to the complete 3D data to obtain a patient head model, and then building a magnetic stimulation point model on the obtained patient head model;
S6, performing first positioning: matching a spatial pose of the patient head model with an actual spatial pose of the patient head to determine a spatial pose of an actual stimulation site of a patient, modeling an operating device, planning a movement path of a mechanical arm, and moving, by the mechanical arm automatically, to a magnetic stimulation point of the patient head according to the planned path for magnetic stimulation treatment; and
S7, performing repeated positioning: after the first positioning is completed, saving magnetic stimulation point information of the patient head and path planning data of the mechanical arm during the first positioning in the patient head model, and during next treatment of the patient, directly invoking the data during the previous positioning to repeatedly position the patient head.

Specifically, in step S2, the illumination compensation algorithm includes the following steps:
S21, analyzing the RGBD image by adopting a contour tracking algorithm to obtain a face contour line of the patient;
S22, mapping the contour line in S21 to the 3D point cloud image of the patient face;
S23, analyzing coordinate values of point clouds on both sides of the contour line along the contour line on the 3D point cloud image, if there is a relatively large sudden change in a height of the coordinate values of the point clouds on the both sides, indicating that a contour is true and effective, and if there is no relatively large sudden change, indicating that the contour is a false contour formed due to shadows caused by too strong light;
S24, adjusting, on the RGBD image, brightness of pixels on both sides of a corresponding position of the false contour to be approximate, that is, adjusting from a dark region to a bright region, to eliminate shadows caused by too strong light;
S25, traversing on the 3D point cloud image to find a region with a relatively large sudden change in the height; and
S26, mapping the region with the relatively large sudden change in the height in S25 to the RGBD image, increasing brightness of pixels in a region with a sudden increase in the height, and decreasing brightness of pixels of a region with a sudden decrease in the height to eliminate an influence caused by too weak light.

Specifically, in step S3, the face detection algorithm is a method based on template matching, a method based on skin color features, or a method based on AdaBoost.

The method based on template matching: the implementation principle of a template matching method is to calculate a correlation coefficient between a target and a preset template by comparing a similarity between the template and a region to be detected. Face detection is to search an image to be detected for a region closest to a face by using a gray-scale template of the face. Compared with other methods for face detection based on features, the face detection method based on template matching is intuitive, simple, and easy to implement, and has strong adaptability, low dependence on image quality, and high robustness.

The method based on skin color features: one of the most significant external features of a face is skin color. The advantage of the skin color is that it does not rely on remaining features of the face, and is not sensitive to changes in a face pose and a face shape. The face can be relatively easily detected by constructing a skin color model that distinguishes the face from other different color backgrounds. However, this method cannot distinguish skin-like objects well, which will cause false detection. In addition, it is relatively sensitive to illumination changes, thereby affecting the accuracy of face detection.

The method based on AdaBoost: first, Haar-like features are adopted to represent a face, and an integral image is used to accelerate a process of Haar-like feature value calcuation; and then AdaBoost is used to screen out best face rectangular features. These features is called weak classifiers, and finally these classifiers are connected in series to form a strong classifier so as to achieve the purpose of detecting a face. In addition, this method is not easily sensitive to the illumination changes.

Specifically, in step S4, the method for spatially positioning the patient face includes the following steps:
S41, obtaining a 3D point cloud image of a face by using an infrared camera in the 3D camera, finding a point of the patient face closest to the camera, that is, the tip of a nose, and obtaining space coordinates (x, y, z) in the camera coordinate system;
S42, using the stable RGBD image obtained in step S2, finding feature points comprising the tip of the nose, a root of the nose, corners of eyes, corners of a mouth, and eyebrows of a human in the image according to a face feature recognition algorithm, and deducing an angle (rx, ry, rz) of the patient face relative to the camera according to left-right symmetry of the face; and
S43, synthesizing the point coordinates and the angle obtained in S41 and S42 into complete 3D space coordinates (x, y, z, rx, ry, rz).

Specifically, in step S6, the planning a movement path of a mechanical arm includes: planning a stimulation sequence of magnetic stimulation points, and planning an optimal path and a movement speed of the mechanical arm to reach each magnetic stimulation point.

Specifically, in the process of the first positioning or repeated positioning, it is required to perform follow-up positioning of the patient head; position information of the patient head each time positioning is completed is recorded in a treatment process, if at a next moment, a distance between positions of the magnetic stimulation point at a current moment and a previous moment exceeds 5 mm due to movement of the patient head, follow-up positioning is started, and if the distance does not exceed 5 mm, follow-up positioning is not started.

Further, the step of the follow-up positioning is: adjusting a spatial pose of the patient head model, so that the spatial pose of the head model is matched with a current actual spatial pose of the patient head, then repositioning a latest magnetic stimulation point on the head model, finally re-planning the movement path of the mechanical arm, and moving the TMS coil to the latest magnetic stimulation point for treatment.

Compared with the prior art, the present invention has the following beneficial effects: (1) in the present invention, RGBD image data of a patient face is acquired by using a 3D camera, and stable RGBD image data and 3D point cloud image data are obtained through calibration, background removal, filtering, and an illumination compensation algorithm, to provide a high-quality data source for face region detection and face feature point detection, and avoid an influence of external factors such as illumination on the face feature point detection, thereby improving the accuracy of 3D pose positioning of a patient head; (2) in the present invention, in a process of the first positioning, an operating device is modeled, and a site where a mechanical arm should be moved is determined through an algorithm; through an algorithm, a stimulation sequence of magnetic stimulation points, an optimal path and a movement speed of the mechanical arm to reach each magnetic stimulation point are planned, thereby ensuring that the mechanical arm will not collide with other devices or other parts of a patient when moving a TMS coil to the magnetic stimulation point.; (3) the present invention also has a repeated positioning function, after the first positioning is completed, magnetic stimulation point information of the patient head and path planning data of the mechanical arm during the first positioning are stored in a patient head model; during next treatment, the patient can directly invoke the data during the previous positioning to position the patient head with one key, thereby improving treatment efficiency; and (4) in the present invention, in the process of first positioning and repeated positioning, follow-up positioning of the patient head can also be performed, during the treatment of the patient, even if the pose of the head changes, fine adjustment of the head model can be quickly performed, so that the spatial pose of the head model is matched with a current actual spatial posture of the patient head, then a latest magnetic stimulation point on the head model is repositioned, finally the movement path of the mechanical arm is re-planned, and the TMS coil is moved to the latest magnetic stimulation point for treatment, thereby enhancing the experience effect of the patient.

### Brief Description of the Drawings

FIG. 1 is a flow schematic block diagram of a TMS positioning and navigation method for TMS treatment which can be implemented using an apparatus according to the present invention;
FIG. 2 is a flow schematic block diagram of an illumination compensation algorithm according to the present invention; and
FIG. 3 is a schematic diagram of a space coordinate system used for obtaining an angle of a human face relative to a camera according to the present invention.

### Detailed Description

The technical solutions of the present invention are clearly and fully described below with reference to the accompanying drawings in the present invention.

### Embodiment 1

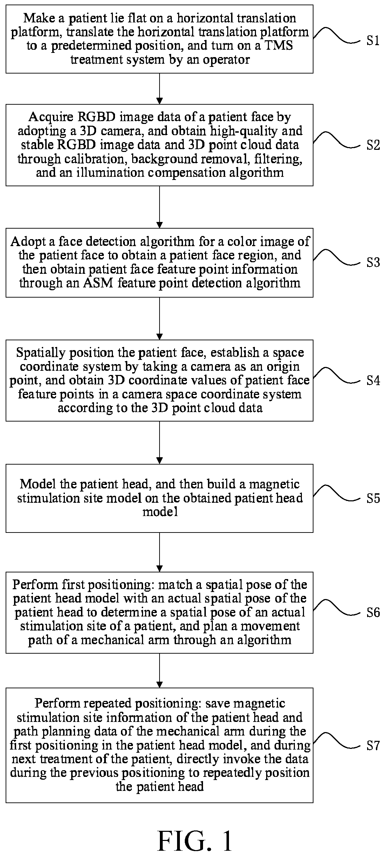

As shown in FIG. 1, the present embodiment provides a TMS positioning and navigation method for TMS treatment, specifically includes the following steps:
S1, making a patient lie flat on a horizontal translation platform, translating the horizontal translation platform to a predetermined position, and turning on a TMS treatment system by an operator;
S2, acquiring RGBD image data of a patient face by adopting a 3D camera, and obtaining stable RGBD image data and 3D point cloud image data through calibration, background removal, filtering, and an illumination compensation algorithm, where the RGBD image data includes an RGBD image, an infrared image, and a depth image;
S3, adopting a face detection algorithm for an RGBD image of the patient face to obtain a patient face region, and then obtaining patient face feature point information through an ASM feature point detection algorithm;
S4, spatially positioning the patient face, establishing a space coordinate system by taking a 3D camera as an origin point, and obtaining 3D coordinate values of patient face feature points in a 3D camera space coordinate system according to the 3D point cloud image data;
S5, modeling the patient head, rotating the 3D camera around the head at a uniform speed or performing photographing at the same time by using multiple cameras to obtain image data of the patient head in all directions, by identifying the face feature point information in the image data in all the directions, calculating matching relationships between images, then obtaining a spatial position relationship between point clouds through an ICP algorithm of the 3D point clouds, integrating all the 3D point cloud image data in scan data to obtain complete 3D data of the patient head, then by taking MNI brain space coordinates commonly used in medicine as a standard, mapping 3D skull data of an MNI space obtained by brain 3D scanning to the complete 3D data to obtain a patient head model, and then building a magnetic stimulation point model on the obtained patient head model;
S6, performing first positioning: matching a spatial pose of the patient head model with an actual spatial pose of the patient head to determine a spatial pose of an actual stimulation site of a patient, modeling an operating device, determining a site where a mechanical arm should be moved through an algorithm, planning a movement path of the mechanical arm through an algorithm, planning a stimulation sequence of magnetic stimulation points, planning an optimal path of the mechanical arm to reach each magnetic stimulation point, and automatically moving, by the mechanical arm, to a magnetic stimulation point of the patient head according to the planned path for magnetic stimulation treatment; and
S7, performing repeated positioning: after the first positioning is completed, saving magnetic stimulation point information of the patient head and path planning data of the mechanical arm during the first positioning in the patient head model, and during next treatment of the patient, directly invoking the data during the previous positioning to repeatedly position the patient head.

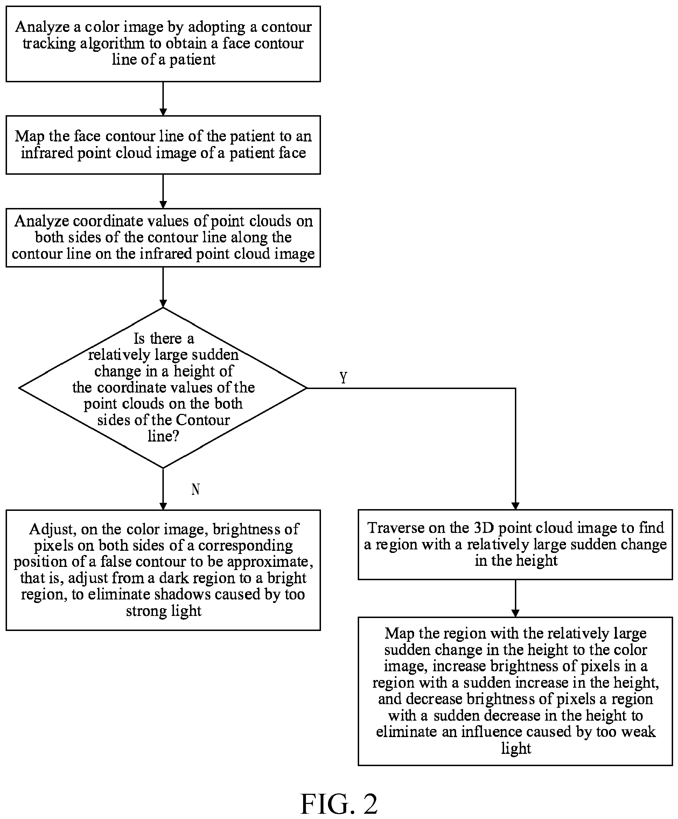

Specifically, as shown in FIG. 2, in step S2, the illumination compensation algorithm includes the following steps:
S21, analyzing the RGBD image by adopting a contour tracking algorithm to obtain a face contour line of the patient;
S22, mapping the contour line in S21 to the 3D point cloud image of the patient face;
S23, analyzing coordinate values of point clouds on both sides of the contour line along the contour line on the 3D point cloud image, if there is a relatively large sudden change in a height of the coordinate values of the point clouds on the both sides, indicating that a contour is true and effective, and if there is no relatively large sudden change, indicating that the contour is a false contour formed due to shadows caused by too strong light;
S24, adjusting, on the RGBD image, brightness of pixels on both sides of a corresponding position of the false contour to be approximate, that is, adjusting from a dark region to a bright region, to eliminate shadows caused by too strong light;
S25, traversing on the 3D point cloud image to find a region with a relatively large sudden change in the height; and
S26, mapping the region with the relatively large sudden change in the height in S25 to the RGBD image, increasing brightness of pixels in a region with a sudden increase in the height, and decreasing brightness of pixels of a region with a sudden decrease in the height to eliminate an influence caused by too weak light.

Specifically, in step S3, the face detection algorithm is a method based on template matching, a method based on skin color features, or a method based on AdaBoost.

The method based on template matching: the implementation principle of a template matching method is to calculate a correlation coefficient between a target and a preset template by comparing a similarity between the template and a region to be detected. Face detection is to search an image to be detected for a region closest to a face by using a gray-scale template of the face. Compared with other methods for face detection based on features, the face detection method based on template matching is intuitive, simple, and easy to implement, and has strong adaptability, low dependence on image quality, and high robustness.

The method based on skin color features: one of the most significant external features of a face is skin color. The advantage of the skin color is that it does not rely on remaining features of the face, and is not sensitive to changes in a face pose and a face shape. The face can be relatively easily detected by constructing a skin color model that distinguishes the face from other different color backgrounds. However, this method cannot distinguish skin-like objects well, which will cause false detection. In addition, it is relatively sensitive to illumination changes, thereby affecting the accuracy of face detection.

The method based on AdaBoost: first, Haar-like features are adopted to represent a face, and an integral image is used to accelerate a process of Haar-like feature value calcuation; and then AdaBoost is used to screen out best face rectangular features. These features is called weak classifiers, and finally these classifiers are connected in series to form a strong classifier so as to achieve the purpose of detecting a face. In addition, this method is not easily sensitive to the illumination changes.

Specifically, in step S4, the method for spatially positioning the patient face includes the following steps:
S41, obtaining a 3D point cloud image of a face by using an infrared camera in the 3D camera, finding a point of the patient face closest to the camera, that is, the tip of a nose, and obtaining space coordinates (x, y, z) in the camera coordinate system;
S42, using the stable RGBD image obtained in step S2, finding feature points comprising the tip of the nose, a root of the nose, corners of eyes, corners of a mouth, and eyebrows of a human in the image according to a face feature recognition algorithm, and deducing an angle (rx, ry, rz) of the patient face relative to the camera according to left-right symmetry of the face; and
S43, synthesizing the point coordinates and the angle obtained in S41 and S42 into complete 3D space coordinates (x, y, z, rx, ry, rz).

Further, in step S41, the (x, y) coordinates of the tip of the nose are obtained by the following method: first, 68 feature points on the face are drawn on Demo through Opencv, and numbered; and then the following model is used:

```
     circle(temp, cvPoint(shapes[0].part(i).x(),
shapes[0].part(i).y()), 3, cv::Scalar(0, 0, 255), -1),
```

where, part(i) represents the ith feature point, and x() and y() are ways to access two-dimensional coordinates of feature points;

Further, a z-axis coordinate of the tip of the nose, that is, a distance from the tip of the nose to the camera, can be obtained though a binocular matching triangulation principle; the triangulation principle refers to that a difference between imaging abscissas of a target point in left and right images (disparity) is inversely proportional to a distance from the target point to an imaging plane: Z=fbd, thereby obtaining the Z-axis coordinate; binocular matching adopts the triangulation principle and is completely based on image processing technology, and matching points are obtained by searching for the same feature points in the two images. The space coordinates of the face in a camera coordinate system are the prior art and are mature, and details are not described herein repeatedly.

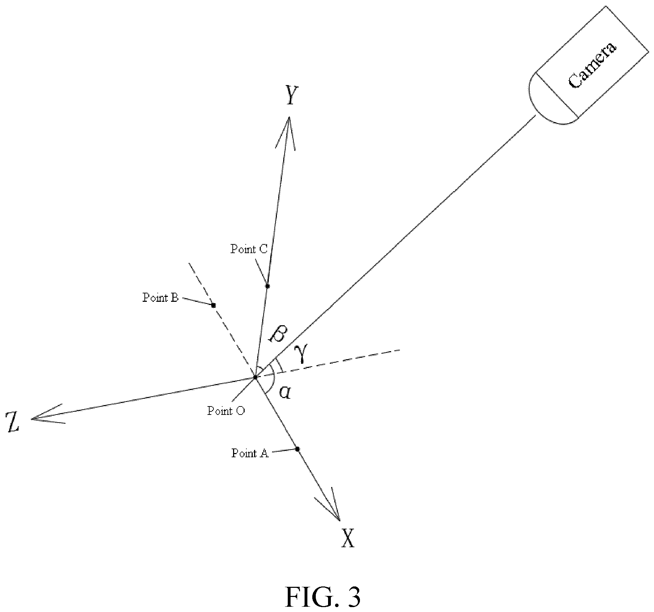

Further, in step S42, the angle (rx, ry, rz) of the patient face relative to the camera is obtained in the following manner:
First, the space coordinate system is constructed, a human head model is constructed according to human face feature points (the tip of the nose, the root of the nose, the corners of the eyes, the corners of the mouth, the eyebrows, etc.) in a picture, then the picture is mapped to the head model, and the face feature points respectively correspond to feature points on the head model; as shown in FIG. 3, on the head model, a plane is created by taking three points, i.e., a left earlobe (point A), a right earlobe (point B), and the tip of the nose (point C) of a human, and a face-based space coordinate system is constructed by using a midpoint point O of point A and point B as an origin of the coordinate system, a direction perpendicular to the plane as an Z axis, a direction of a connection line between point A and B as an X axis, and a direction of a connection line between point C and point O as an Y axis, where the X axis is perpendicular to the Y axis; and then the angle (rx, ry, rz) of the human face relative to the camera can be deduced by calculating included angles α, β, and y between the connection line from point O to the camera and the X axis, the Y axis, and the Z axis.

Specifically, in step S5, modeling a head needs to acquire 3D scan data of a head of a patient by a 3D camera. Each time the 3D camera performs photographing, a color image, a depth image, and a 3D point cloud image are generated. The three images are generated at the same time, and thus, points on the images have a fixed corresponding relationship. The corresponding relationship is known and is obtained through calibration of the camera. 3D scanning is to capture a series of images around a head of a patient, and then stitch the images into a complete image. Image stitching involves finding the same parts of two images and matching same. No 3D point cloud for hair can be obtained in a 3D camera, but 3D data of the skull (without hair) is needed in a medical treatment head model. Therefore, a patient needs to wear a specific cap during scanning for a head model. In order to make the matching more accurate, some mark points are usually provided on the cap. 3D scanning ultimately needs to stitch 3D point clouds. The rotation and translation relationship between point clouds of all images is needed during stitching. The stitching of point clouds mainly relies on an ICP algorithm.

Further, stitching of point clouds includes the follow steps.

At S51, "key points" are first calculated in a color image through cv::FeatureDetector and cv::DescriptorExtractor in OpenCV, "descriptors" of pixels around the key points are calculated, then the above descriptors are matched using cv::DMatch, and then a SolvePnPRansac function in OpenCV is called to solve PnP to obtain displacement and rotation information of two images.

At S52, point cloud data of the two images are calculated by using the displacement and rotation information calculated above as a result of initial coarse matching of the ICP algorithm to obtain more precise displacement and rotation data.

At S53, a displacement and rotation matrix is obtained using the above displacement and rotation data, all points in a previous point cloud image are rotated and translated, and a new point cloud calculated is added to a current point cloud image to obtain a larger point cloud and complete integration of the two point clouds.

At S54, steps S51 to S53 are repeated, all point cloud images are integrated into a larger point cloud image, then filtering and smoothing processing is performed on the point cloud image, sampling is performed to reduce the number of points, and fitting is performed to obtain 3D curved surface data, so as to obtain complete 3D data of the head of the patient.

Specifically, in step S6, the spatial pose of the patient head model is matched with the actual spatial pose of the patient head. The specific matching method is: in the treatment process, the 3D image captured by the 3D camera in real time has only face information of the patient but no head information, and thus, the head model constructed in S5 needs to be aligned in position with face data captured in real time. Requirements of real-time detection cannot be satisfied due to a large calculation amount of an ICP algorithm. The position alignment method includes: first marking face feature points (corners of eyes, a tip of a nose, etc.) for alignment in a head model, then automatically identifying the face feature points in a real-time image, and calculating a conversion relationship between the real-time image and the head model through feature point matching, calculating a position of the head model in space, and then calculating position coordinates of a magnetic stimulation point on the head model in space.

Further, the modeling an operating device specifically includes modeling the mechanical arm, the 3D camera, and the TMS coil, placing the built model and the patient head model in the same space coordinate (camera space coordinate system), then selecting a target to be magnetically stimulated on the head model, calculating the best path of the TMS coil to reach the target point in the space model, and ensuring that there is no collision during movement.

Further, a movement path planning algorithm for a mechanical arm is relatively complicated in general, and since the models, obstacle, and path in the present embodiment are all known, a manual path planning method is adopted. A straight path is used at a position far away from the head model, and an arc path is used near the head model, so as to move the TMS coil around the head to a next magnetic stimulation target to be magnetically stimulated. Since 3D data of the head model is known, head model data can be enlarged to leave a safe distance for running, and a shortest arc path between two points on the head model can be calculated.

### Embodiment 2

The present embodiment provides a TMS positioning and navigation method for TMS treatment, and differs from the above embodiment in that the TMS positioning and navigation method according to the present embodiment further includes a following position method.

Specifically, in the process of first positioning or repeated positioning, it is required to perform follow-up positioning of a patient head. In a treatment process, position information of the patient head every time the positioning is completed will be recorded. If a distance between a current target position and a target position at a previous moment exceeds 5 mm due to motion of the patient head, a follow-up positioning action is started, fine adjustment of the head model and mechanical arm navigation is performed through an algorithm, a correct magnetic stimulation point is repositioned, a motion path of the mechanical arm is re-calculated, and a coil is moved to a new target position; if the distance does not exceed 5 mm, the follow-up positioning action will not be started. If the patient turns a lot, the following of the camera and a mechanical arm are paused, and coil magnetic stimulation is paused; if the patient is not within an adjustable range of the camera or leaves, mechanical arm and coil magnetic stimulation actions are stopped.

### Embodiment 3

The present embodiment further provides a TMS positioning and navigation device for TMS treatment, including a lying bed, a headrest, a 3D camera, and a mechanical arm. During treatment, a patient lies flat on the lying bed, and rests the head on the headrest, 3D data of the patient head is acquired through he 3D camera, in combination with an algorithm, a magnetic stimulation point on the patient head is precisely positioned, and then the mechanical arm is controlled to move a TMS coil to the magnetic stimulation point to treat the patient.

The lying bed can be moved forwards and backwards, and is configured to adjust a relative position of the patient head and the camera.

The headrest mainly functions as a bracket, and supporting sites are a skull and a neck, are configured to limit movement of the patient without causing discomfort to the patient, and does not affect magnetic stimulation of the back of the head.

The 3D camera adopts an existing camera, and multiple 3D cameras can be used to acquire images of the patient head from multiple angles, or one camera can be combined with other devices to move, rotate and scan the patient head to acquire the 3D data of the patient head.

The location where the mechanical arm and the coil are combined is provided with a pressure sensor, and the contact between the coil and a patient brain will not cause loose fit or excessive pressure, which can effectively improve the experience of the patient.

## Claims

1. A Transcranial Magnetic Stimulation, TMS, positioning and navigation apparatus for TMS treatment comprising: a lying bed, a headrest, a 3D camera and a mechanical arm,
wherein the lying bed and the headrest are configured that during TMS treatment of a user, the user lies flat on the lying bed and the user head rests on the headrest, and wherein further the lying bed is configured to be moved forwards and backwards, and to adjust a relative position of the user head and the 3D camera,
the TMS positioning and navigation apparatus being configured to perform steps of:
S1, translate the user to a predetermined position through the lying bed;
S2, scan the head of the user through the 3D camera to obtain 3D model data of the head of the user, and construct a 3D model according to the 3D model data of the head of the user;
S3, perform first positioning: detect positions of the head of the user and a TMS coil, matching the 3D model with the head of the user to obtain a precise location to be stimulated, plan, by a controller, a movement path of the mechanical arm according to the positions of the head of the user and the TMS coil, and control the mechanical arm to precisely move the TMS coil to the head of the user;
S4, perform follow-up positioning: if the position of the head of the user changes, detect a new position of the head of the user in real time, and match the 3D model with the new position to obtain a precise location to be stimulated; and
S5, perform repeated positioning: after the first positioning is completed, save treatment location information of the user head and path planning data of the mechanical arm during the first positioning in user head model data, and during next treatment of the user, directly invoke the data during the previous positioning to repeatedly position the user head.

2. The TMS positioning and navigation apparatus according to claim 1, wherein the step S2 comprises:
S21, acquire RGBD image data of a user face by the 3D camera, and obtain stable RGBD image data and 3D point cloud image data through calibration, background removal, filtering, and an illumination compensation algorithm;
S22, obtain user face feature point information: processing an RGBD image of the patient face by adopting a face detection algorithm to obtain a user face region, and then obtain user face feature point information through an ASM feature point detection algorithm;
S23, spatially position the user face, establishing a space coordinate system by taking the 3D camera as an origin point, and obtaining 3D coordinate values of a user face feature point in a 3D camera space coordinate system according to the 3D point cloud image data; and
S24, model the user head, rotating the 3D camera around the head at a uniform speed or performing photographing at the same time by using multiple 3D cameras to obtain image data of the user head in all directions, by identifying the face feature point information in the image data in all the directions, calculating matching relationships between images in all the directions, then obtaining a spatial position relationship between point clouds through an ICP algorithm of the 3D point clouds, and integrating all the 3D point cloud image data in scan data to obtain complete 3D data of the user head; and then use MNI brain space coordinates as a standard, map 3D skull data of an MNI space to the complete 3D data to obtain a user head model, and then build a magnetic stimulation point model on the user head model.

3. The TMS positioning and navigation apparatus according to claim 2, wherein the illumination compensation algorithm is configured to perform the steps:
S211, analyze the RGBD image by adopting a contour tracking algorithm to obtain a face contour line of the user;
S212, map the contour line in S211 to the 3D point cloud image of the user face;
S213, analyze coordinate values of point clouds on both sides of the contour line along the contour line on the 3D point cloud image, if there is a relatively large sudden change in a height of the coordinate values of the point clouds on the both sides, indicate that a contour is true and effective, and if there is no relatively large sudden change, indicate that the contour is a false contour formed due to shadows caused by too strong light;
S214, adjust, on the RGBD image, brightness of pixels on both sides of a corresponding position of the false contour to be approximate, that is, adjust from a dark region to a bright region, to eliminate shadows caused by too strong light;
S215, traverse on the 3D point cloud image to find a region with a relatively large sudden change in the height; and
S216, map the region with the relatively large sudden change in the height in S215 to the RGBD image, increase brightness of pixels in a region with a sudden increase in the height, and decrease brightness of pixels of a region with a sudden decrease in the height to eliminate an influence caused by too weak light.

4. The TMS positioning and navigation apparatus according to claim 2, wherein the face detection algorithm is a method based on template matching, a method based on skin color features, or a method based on AdaBoost.

5. The TMS positioning and navigation apparatus according to claim 2, wherein the step S23 comprises:
S231, obtain a 3D point cloud image of a face by using the 3D camera, find a point of the user face closest to the camera, that is, the tip of a nose, and obtain space coordinates (x, y, z) in the camera coordinate system;
S232, use the stable RGBD image obtained in step S21, find feature points comprising the tip of the nose, a root of the nose, corners of eyes, corners of a mouth, and eyebrows of a human in the image according to a face feature recognition algorithm, and deduce an angle (rx, ry, rz) of the user face relative to the camera according to left-right symmetry of the face; and
S233, synthesize the point coordinates and the angle obtained in S231 and S232 into complete 3D space coordinates (x, y, z, rx, ry, rz).

6. The TMS positioning and navigation apparatus according to claim 1, wherein the step S3 comprises: plan a stimulation sequence of magnetic stimulation points, and plan an optimal path and a movement speed of the mechanical arm to reach each magnetic stimulation point.

7. The TMS positioning and navigation apparatus according to claim 1, wherein the step S4 comprises:
record position information of a magnetic stimulation point of the user head each time positioning is completed in a treatment process, if at a next moment, a distance between positions of the magnetic stimulation point at a current moment and a previous moment exceeds 5 mm due to movement of the user head, start follow-up positioning, and if the distance does not exceed 5 mm, not start follow-up positioning;
after starting the follow-up positioning, first adjust a spatial pose of the user head model, so that the spatial pose of the head model is matched with a current actual spatial pose of the user head, then reposition a latest magnetic stimulation point on the head model, finally re-plan the movement path of the mechanical arm, and move the TMS coil to the latest magnetic stimulation point for treatment.

## Patentansprüche

1. Positionierungs- und Navigationsvorrichtung für transkraniale Magnetstimulation, TMS, zur TMS-Behandlung, umfassend: eine Liege, eine Kopfstütze, eine 3D-Kamera und einen mechanischen Arm,
wobei die Liege und die Kopfstütze so konfiguriert sind, dass ein Benutzer während der TMS-Behandlung flach auf der Liege liegt und der Kopf des Benutzers auf der Kopfstütze ruht, und wobei die Liege ferner dazu konfiguriert ist, vor und zurück bewegt zu werden und eine relative Position des Kopfs des Benutzers und der 3D-Kamera einzustellen,
wobei die TMS-Positionierungs- und Navigationsvorrichtung zum Durchführen der folgenden Schritte konfiguriert ist:
S1 Verlagern des Benutzers in eine vorgegebene Position durch die Liege;
S2 Abtasten des Kopfs des Benutzers durch die 3D-Kamera, um 3D-Modelldaten des Kopfs des Benutzers zu erlangen und ein 3D-Modell gemäß den 3D-Modelldaten des Kopfs des Benutzers zu erstellen;
S3 Durchführen einer ersten Positionierung durch Erkennen von Positionen des Kopfs des Benutzers und einer TMS-Spule, Anpassen des 3D-Modells an den Kopf des Benutzers, um eine genaue Stelle für die Stimulation zu erlangen, Planen eines Bewegungspfads des mechanischen Arms gemäß den Positionen des Kopfs des Benutzers und der TMS-Spule durch eine Steuereinrichtung und Steuern des mechanischen Arms, um die TMS-Spule präzise zu dem Kopf des Benutzers zu bewegen;
S4 Durchführen einer Nachpositionierung, wenn sich die Position des Kopfs des Benutzers ändert, durch Erkennen einer neuen Position des Kopfs des Benutzers in Echtzeit und Anpassen des 3D-Modells an die neue Position, um eine genaue Stelle für die Stimulation zu erlangen; und
S5 Durchführen einer wiederholten Positionierung nach dem Abschluss der ersten Positionierung durch Speichern der Behandlungsstelleninformationen des Benutzerkopfs und der Pfadplanungsdaten des mechanischen Arms aus der ersten Positionierung in Benutzerkopfmodelldaten, und bei der nächsten Behandlung des Benutzers direktes Aufrufen der Daten aus der vorangehenden Positionierung, um den Benutzerkopf erneut zu positionieren.

2. TMS-Positionierungs- und Navigationsvorrichtung nach Anspruch 1, wobei Schritt S2 umfasst:
S21 Erfassen von RGBD-Bilddaten des Gesichts eines Benutzers durch die 3D-Kamera und Erlangen stabiler RGBD-Bilddaten und 3D-Punktwolkenbilddaten durch Kalibration, Hintergrundentfernung, Filterung und einen Beleuchtungskompensationsalgorithmus;
S22 Erlangen von Gesichtsmerkmalspunktinformationen des Benutzers durch Verarbeiten eines RGBD-Bilds des Gesichts des Benutzers durch Anwenden eines Gesichtserkennungsalgorithmus, um einen Bereich des Gesichts des Benutzers zu erlangen, und dann Erlangen der Gesichtsmerkmalspunktinformationen des Benutzers durch einen ASM-Merkmalspunkterkennungsalgorithmus;
S23 räumliches Positionieren des Gesichts des Benutzers, Erstellen eines Raumkoordinatensystems mit der 3D-Kamera als Ursprungspunkt und Erlangen von 3D-Koordinatenwerten eines Merkmalspunkts des Gesichts des Benutzers in einem 3D-Kameraraumkoordinatensystem gemäß den 3D-Punktwolken-Bilddaten; und
S24 Modellieren des Kopfs des Benutzers, Rotierenlassen der 3D-Kamera um den Kopf mit gleichmäßiger Geschwindigkeit oder gleichzeitiges Fotografieren unter Verwendung mehrerer 3D-Kameras zum Erlangen von Bilddaten des Kopfs des Benutzers aus allen Richtungen durch Identifizieren der Gesichtsmerkmalspunktinformationen in den Bilddaten aus allen Richtungen, Berechnen von Übereinstimmungsbeziehungen zwischen Bildern aus allen Richtungen und dann Erlangen einer räumlichen Positionsbeziehung zwischen Punktwolken durch einen ICP-Algorithmus der 3D-Punktwolken und Integrieren aller 3D-Punktwolken-Bilddaten in Abtastdaten, um vollständige 3D-Daten des Kopfs des Benutzers zu erlangen; und daraufhin Verwenden von MNI-Gehirnraumkoordinaten als Standard, Zuordnen von 3D-Schädeldaten eines MNI-Raums zum Vervollständigen der 3D-Daten zum Erlangen eines Kopfmodells des Benutzers und sodann Erstellen eines Modells mit Magnetstimulationspunkten am Kopfmodell des Benutzers.

3. TMS-Positionierungs- und Navigationsvorrichtung nach Anspruch 2, wobei der Beleuchtungskompensationsalgorithmus zum Durchführen der folgenden Schritte konfiguriert ist:
S211 Analysieren des RGBD-Bilds durch Anwenden eines Konturverfolgungsalgorithmus, um eine Gesichtskonturlinie des Benutzers zu erlangen;
S212 Abbilden der Konturlinie aus S211 auf das 3D-Punktwolkenbild des Gesichts des Benutzers;
S213 Analysieren von Koordinatenwerten von Punktwolken auf beiden Seiten der Konturlinie an der Konturlinie in dem 3D-Punktwolkenbild, und wenn eine relativ große plötzliche Veränderung der Höhe der Koordinatenwerte der Punktwolken auf beiden Seiten vorliegt, Angeben, dass eine Kontur korrekt und wirksam ist, und wenn keine relativ große plötzliche Veränderung vorliegt, Angeben, dass die Kontur eine falsche Kontur aufgrund von Schatten durch zu starkes Licht ist;
S214 Anpassen der Helligkeit von Pixeln auf beiden Seiten einer entsprechenden Position der falschen Kontur in dem RGBD-Bild zur Annäherung, das heißt, Anpassen von einem dunklen Bereich zu einem hellen Bereich, um Schatten durch zu starkes Licht zu beseitigen;
S215 Durchlaufen des 3D-Punktwolkenbilds, um einen Bereich mit einer relativ großen plötzlichen Veränderung der Höhe zu finden; und
S216 Abbilden des Bereichs mit der relativ großen plötzlichen Veränderung aus S215 auf das RGBD-Bild, Erhöhen der Helligkeit von Pixeln in einem Bereich mit einer plötzlichen Zunahme der Höhe und Verringern der Helligkeit von Pixeln eines Bereichs mit einer plötzlichen Abnahme der Höhe, um Einflüsse aufgrund von zu schwachem Licht zu beseitigen.

4. TMS-Positionierungs- und Navigationsvorrichtung nach Anspruch 2, wobei der Gesichtserkennungsalgorithmus ein auf Vorlagenabgleich beruhendes Verfahren, ein auf Hautfarbenmerkmalen beruhendes Verfahren oder ein auf AdaBoost beruhendes Verfahren ist.

5. TMS-Positionierungs- und Navigationsvorrichtung nach Anspruch 2, wobei Schritt S23 umfasst:
S231 Erlangen eines 3D-Punktwolkenbilds eines Gesichts unter Verwendung der 3D-Kamera, Auffinden eines Punkts im Gesicht des Benutzers, der der Kamera am nächsten ist, also die Spitze einer Nase, und Erlangen von Raumkoordinaten (x, y, z) in dem Kamerakoordinatensystem;
S232 Verwenden des stabilen RGBD-Bilds aus Schritt S21, Auffinden von Merkmalspunkte, die die Nasenspitze, die Nasenwurzel, Augenwinkel, Mundwinkel und Augenbrauen eines Menschen in dem Bild umfassen, gemäß einem Gesichtsmerkmalserkennungsalgorithmus, und Herleiten eines Winkels (rx, ry, rz) des Gesichts des Benutzers relativ zu der Kamera gemäß Links-rechts-Symmetrie des Gesichts; und
S233 Zusammenführen der Punktkoordinaten und des Winkels aus S231 und S232 zu vollständigen 3D-Raumkoordinaten (x, y, z, rx, ry, rz).

6. TMS-Positionierungs- und Navigationsvorrichtung nach Anspruch 1, wobei Schritt S3 umfasst: Planen einer Stimulationssequenz von Magnetstimulationspunkten und Planen eines optimalen Pfads und einer Bewegungsgeschwindigkeit des mechanischen Arms zum Erreichen jedes Magnetstimulationspunkts.

7. TMS-Positionierungs- und Navigationsvorrichtung nach Anspruch 1, wobei Schritt S4 umfasst:
Aufzeichnen von Positionsinformationen eines Magnetstimulationspunkts des Kopfs des Benutzers nach Abschluss jeder Positionierung in einem Behandlungsvorgang, und wenn zu einem nächsten Zeitpunkt ein Abstand zwischen Positionen des Magnetstimulationspunkts zum aktuellen Zeitpunkt und einem vorhergehenden Zeitpunkt aufgrund einer Bewegung des Kopfs des Benutzers größer als 5 mm ist, Starten der Nachpositionierung, und wenn der Abstand nicht größer als 5 mm ist, kein Starten der Nachpositionierung;
nach dem Starten der Nachpositionierung erstes Einstellen einer räumlichen Stellung des Benutzerkopfmodells, sodass die räumliche Stellung des Kopfmodells mit einer aktuellen tatsächlichen räumlichen Stellung des Kopfs des Benutzers übereinstimmt, dann erneutes Positionieren eines letzten Magnetstimulationspunkts an dem Kopfmodel und schließlich erneutes Planen des Bewegungspfads des mechanischen Arms und Bewegen der TMS-Spule an den letzten Magnetstimulationspunkt zur Behandlung.

## Revendications

1. Un dispositif de navigation et positionnement pour un traitement de stimulation magnétique transcrânienne, TMS, comprenant : un lit pour s'étendre, un repose-tête, une caméra 3D et un bras mécanique,
dans lequel le lit pour s'étendre et le repose-tête sont configurés afin que lors du traitement TMS d'un utilisateur, l'utilisateur soit entièrement étendu sur le lit pour s'étendre et la tête de l'utilisateur repose sur le repose-tête, et dans lequel en outre le lit pour s'étende est configuré pour être déplacé vers l'avant et vers l'arrière, et pour ajuster la position relative de la tête de l'utilisateur et la caméra 3D, le dispositif de navigation et positionnement TMS étant configuré pour réaliser des étapes :
S1, translater l'utilisateur à une position prédéterminée à l'aide du lit pour s'étendre ;
S2, scanner la tête de l'utilisateur à l'aide de la caméra 3D pour obtenir des données de modèle 3D de la tête de l'utilisateur, et construire un modèle 3D en fonction des données de modèle 3D de la tête de l'utilisateur ;
S3, réaliser un premier positionnement : détecter les positions de la tête de l'utilisateur et d'une bobine TMS, ajustée au modèle 3D avec la tête de l'utilisateur pour obtenir une localisation précise à stimuler, planifier, par contrôleur, un chemin de mouvement du bras mécanique en fonction des positions de la tête de l'utilisateur et la bobine TMS, et commander le bras mécanique pour déplacer de façon précise la bobine TMS vers la tête de l'utilisateur ;
S4, réaliser un suivi de positionnement : si la position de la tête de l'utilisateur change, détecter la nouvelle position de la tête de l'utilisateur en temps réel, et ajuster le modèle 3D à la nouvelle position pour obtenir une localisation précise à stimuler ; et
S5, réaliser des positionnements répétés : une fois le premier positionnement achevé, sauvegarder les informations de localisation de traitement de la tête de l'utilisateur et les données de planification de chemin du bras mécanique durant le premier positionnement dans les données de modèle de tête de l'utilisateur, et durant le traitement suivant de l'utilisateur, appeler directement les données du positionnement précédent afin de positionner de façon répétée la tête de l'utilisateur.

2. Un dispositif de navigation et positionnement pour un traitement de TMS selon la revendication 1, dans lequel l'étape S2 comprend :
S21, acquérir les données d'image RGBD du visage d'un utilisateur à l'aide d'une caméra 3D, et obtenir des données d'image RGBD stables et des données d'image de nuages de points 3D via calibrage, élimination de l'arrière-plan, filtrage, et un algorithme de compensation d'éclairage ;
S22, obtenir les informations des points caractéristiques du visage de l'utilisateur : traiter l'image RGBD du visage du patient en adoptant un algorithme de détection de visage pour obtenir la région du visage de l'utilisateur, puis obtenir les informations de points caractéristiques du visage de l'utilisateur via un algorithme de détection des points caractéristiques ASM ;
S23, positionnement spatial du visage de l'utilisateur, établir un système de coordonnées spatiales en prenant la caméra 3D comme point d'origine, et obtenir les valeurs des coordonnées 3D des points caractéristiques du visage de l'utilisateur dans un système de coordonnées spatiale de caméra 3D en fonction des données d'images de nuage de points 3D ; et
S24, modéliser la tête de l'utilisateur, faire pivoter la caméra 3D autour de la tête à vitesse homogène ou réaliser des photographies au même moment en utilisant de multiples caméras 3D pour obtenir des données d'images de la tête de l'utilisateur dans toutes les directions, en identifiant les informations de points caractéristiques du visage dans les données d'images dans toutes les directions, calculer les relations de correspondance entre les images dans toutes les directions, puis obtenir une relation de position spatiale entre les nuages de points via un algorithme ICP des nuages de points 3D, et intégrer toutes les données d'images de nuages de points 3D dans les données de scan pour obtenir les données 3D complètes de la tête de l'utilisateur ; puis utiliser les coordonnées spatiales cérébrales MNI comme standard, cartographier les données crâniennes 3D d'un espace MNI sur les données 3D complètes pour obtenir le modèle de tête de l'utilisateur, puis construire un modèle de points de stimulation magnétique sur le modèle de tête de l'utilisateur.

3. Un dispositif de navigation et positionnement pour un traitement de TMS selon la revendication 2, dans lequel l'algorithme de compensation d'éclairage est configurée pour réaliser les étapes :
S211, analyser l'image RGBD en adoptant un algorithme de suivi de contour pour obtenir une ligne de contour de visage de l'utilisateur ;
S212, cartographier la ligne de contour en S211 sur l'image de nuage de points 3D du visage de l'utilisateur ;
S213, analyser les valeurs des coordonnées des nuages de points sur les deux côtés de la ligne de contour le long de la ligne de contour sur l'image de nuages de points 3D, en cas de changement soudain relativement important dans la hauteur des valeurs des coordonnées des nuages de points sur les deux côtés, indiquer qu'un contour est vrai et efficace, et s'il n'y a pas de changement soudain relativement important, indiquer que le contour est un faux contour formé en raison d'ombres provoquées par une lumière trop forte ;
S214, ajuster, sur l'image RGBD, la luminosité des pixels sur les deux côtés d'une position correspondante du faux contour de façon approchante, c'est-à-dire, ajuster d'une région sombre à une région lumineuse, pour éliminer les ombres provoquées par une lumière trop forte ;
S215, traverser sur l'image de nuage de points 3D afin de trouver une région dotée d'un changement soudain relativement important sur la hauteur ; et
S216, cartographier la région à changement soudain relativement important dans la hauteur en S215 sur l'image RGBD, augmenter la luminosité des pixels dans une région à changement soudain relativement important dans la hauteur, et réduire la luminosité des pixels d'une région à décroissance soudaine relativement importante dans la hauteur pour éliminer l'influence provoquée par une lumière trop faible.

4. Un dispositif de navigation et positionnement pour un traitement de TMS selon la revendication 2, dans lequel l'algorithme de détection de visage est un procédé basé sur une correspondance de modèles, un procédé basé sur les caractéristiques de couleur de peau, ou un procédé basé sur AdaBoost.

5. Un dispositif de navigation et positionnement pour un traitement de TMS selon la revendication 2, dans lequel l'étape S23 comprend :
S231, obtenir une image de nuage de points 3D d'un visage en utilisant la caméra 3D, trouver le point du visage de l'utilisateur le plus près de la caméra, c'est-à-dire, le bout du nez, et obtenir les coordonnées spatiales (x, y, z) dans le système de coordonnées de la caméra ;
S232, utiliser l'image RGBD stable obtenue à l'étape S21, trouver les points caractéristiques comprenant le bout du nez, la racine du nez, les coins des yeux, les coins de la bouche, et les sourcils de l'humain sur l'image en fonction de l'algorithme de reconnaissance des caractéristiques faciales, et déduire l'angle (rx, ry, rz) du visage de l'utilisateur par rapport à la caméra en fonction de la symétrie gauche-droite du visage ; et
S233, synthétiser les coordonnées des points et angles obtenus en S231 et S232 en des coordonnées spatiales 3D complètes (x, y, z, rx, ry, rz).

6. Un dispositif de navigation et positionnement pour un traitement de TMS selon la revendication 1, dans lequel l'étape S3 comprend : planifier une séquence de stimulation de points de stimulation magnétique, et planifier le chemin optimal et la vitesse de mouvement du bras mécanique afin d'atteindre chaque point de stimulation magnétique.

7. Un dispositif de navigation et positionnement pour un traitement de TMS selon la revendication 1, dans lequel l'étape S4 comprend :
enregistrer les informations de position d'un point de stimulation magnétique de la tête de l'utilisateur chaque fois que le positionnement est achevé dans une procédure de traitement, si à un moment suivant, la distance entre les positions de point de stimulation magnétique à un moment actuel et un moment précédent dépasse 5 mm en raison d'un mouvement de tête de l'utilisateur, commencer le positionnement de suivi, et si la distance ne dépasse pas 5 mm, ne pas commencer le positionnement de suivi ;
après avoir commencé le positionnement de suivi, ajuster d'abord la pose spatiale du modèle de tête de l'utilisateur, de sorte que la pose spatiale du modèle de tête de l'utilisateur corresponde à la pose spatiale du modèle de tête de l'utilisateur en cours, puis repositionner le tout dernier point de stimulation magnétique sur le modèle de tête, enfin replanifier le chemin de mouvement du bras mécanique, et déplacer la bobine TMS sur le tout dernier point de stimulation magnétique pour traitement.
